# EUROPÄISCHE PATENTANMELDUNG

(11) **EP 0 607 606 A1**
(43) Veröffentlichungstag der Anmeldung: **27.07.1994**
(21) Anmeldenummer: 93120810.2
(22) Anmeldetag: 23.12.1993
(51) Int. Cl.: A61F 13/06

(54) **Bandage zur Fixierung des Sprunggelenks**

(30) Priorität: 18.01.1993 DE 4301145
(71) Anmelder: Kessler, Sigurd, Dr., D-82178 Puchheim (DE)
(72) Erfinder: Kessler, Sigurd, Dr., D-82178 Puchheim (DE)
(74) Vertreter: Finsterwald, Manfred, Dipl.-Ing., Dipl.-Wirtsch.-Ing.

(57) **Zusammenfassung**

Es wird eine Bandage zur Fixierung des Sprunggelenks beschrieben, die aus einer dem Mittelfußbereich zugeordneten Haltebandage, einer den Knöchelbereich umschlingenden Ringbandage und zumindest einem diese beiden Bandagen miteinander verbindenden, spannbaren Zugband besteht.

## Beschreibung

Die Erfindung betrifft eine Bandage zur Fixierung des Sprunggelenks.

Verletzungen von Bändern im Bereich des Sprunggelenks treten in der Praxis sehr häufig auf und sind im Regelfall eine Folge eines Umknickens des Fußes. Dabei kommt es zu Dehnungen, Rissen oder Einrissen von Bändern, die sehr schmerzhaft sind. Besonders häufig betroffen ist dabei das sich in Richtung des Vorderfußes zwischen dem Wadenbein und dem Sprungbein erstreckende Band.

Um eine Heilung der Verletzung zu ermöglichen und den auftretenden Schmerzen entgegenzuwirken wird das Sprunggelenk durch Anlegen eines Gipses, Anbandagieren einer L-Schiene oder einen sonstigen Stillhalteverband ruhiggestellt. Nachteilig ist dabei, daß durch diese Ruhigstellung der betroffene Patient sein normales Schuhwerk für längere Zeit nicht mehr benutzen kann und dadurch entsprechend behindert ist, und daß überdies im Regelfall das gesamte Sprunggelenk immobilisiert wird, was nach dem Ausheilen der Verletzung aufwendige krankengymnastische Beweglichkeitsübungen erforderlich macht.

Aufgabe der Erfindung ist es, eine Sprunggelenkbandage zu schaffen, die eine gezielte, das durch die Verletzung betroffene Band entlastende Ruhigstellung ermöglicht und dabei im normalen Schuh getragen werden kann. Außerdem soll diese Bandage einfach, schnell und korrekt anlegbar sein.

Gelöst wird diese Aufgabe nach der Erfindung im wesentlichen durch eine den Mittelfußbereich umspannende Haltebandage und ein mit dieser Haltebandage fußaußenseitig verbundenes, sich zumindest im wesentlichen in Richtung des Außenknöchels erstreckendes Zugband, das mittels einer den Knöchelbereich umschlingenden, am Zugbandende angreifenden Ringbandage spannbar ist.

Wesentlich für die Erfindung ist somit, daß ein Zugband vorgesehen ist, das etwa entsprechend dem beschädigten oder gerissenen Band verläuft und praktisch zwischen zwei durch die Haltebandage und die Ringbandage geschaffenen Festpunkten gespannt werden kann. Auf diese Weise ist es möglich, den äußeren Fußrand in insbesondere leicht angehobener Stellung zu fixieren, damit das geschädigte Band zu entlasten und außerdem einer Bewegung des Sprungbeins nach vorne entgegenzuwirken. Da sämtliche Bestandteile der Bandage flach ausgebildet sind, läßt sich die Bandage problemfrei auch im Schuh tragen.

Eine besonders vorteilhafte Möglichkeit der Schaffung eines fußaußenseitig gelegenen Befestigungs-Fixpunktes besteht darin, ein den Fußaußenrand nach oben und unten übergreifendes Formteil zu verwenden, das von der Haltebandage umspannt wird. Dabei wird dafür gesorgt, daß die Haltebandage bezüglich dieses Formteils nicht verrutschen kann, und auf diese Weise wird fußaußenseitig ein Festpunkt geschaffen, der eine optimale Anlenkstelle für das Zugband darstellt.

Das Spannen des Zugbandes kann entweder über die Ringbandage oder auch dadurch erfolgen, daß die Ringbandage lediglich zur Schaffung eines zweiten Fixpunktes verwendet und das Zugband selbst zwischen den beiden Fixpunkten spannbar ausgebildet wird.

Weitere vorteilhafte Ausgestaltungen der Erfindung sind in den Unteransprüchen angegeben.

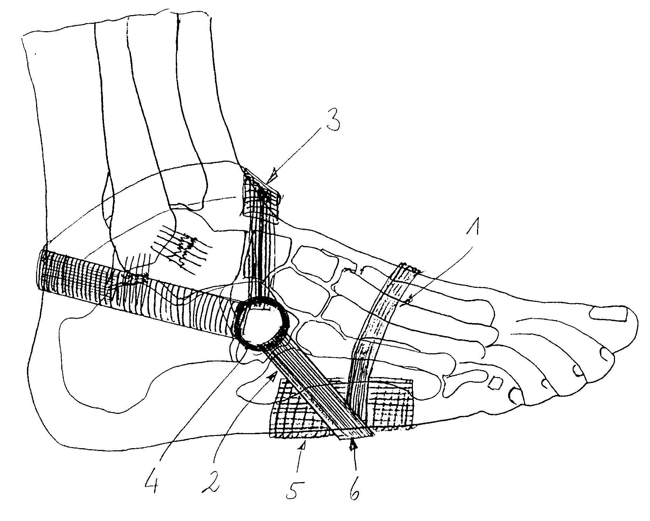

Ein Ausführungsbeispiel der Erfindung wird nachfolgend unter Bezugnahme auf die Zeichnung erläutert; die einzige Figur der Zeichnung zeigt eine schematische Darstellung einer Bandage gemäß der Erfindung im angelegten Zustand.

Die Zeichnung zeigt einen durch eine erfindungsgemäße Bandage ruhiggestellten Fuß, wobei die Lage und der Verlauf geschädigter Bänder, insbesondere des sich zwischen Wadenbein und Sprungbein erstreckenden Bandes dargestellt ist.

Durch die Bandage muß erreicht werden, daß das betroffene Band, das gedehnt, angerissen oder durchgerissen sein kann, entlastet wird und demgemäß praktisch keine Zugkräfte auf dieses Band einwirken können.

Dieses Ziel wird dadurch erreicht, daß zwischen zwei durch eine Haltebandage 1 und eine Ringbandage 3 geschaffenen Fixpunkten ein Zugband 2 vorgesehen ist, das zumindest in etwa in Richtung des beschädigten Bandes verläuft und so gespannt werden kann, daß der äußere Fußrand zumindest in leicht angehobener Stellung fixiert wird, was zur Folge hat, daß praktisch keine Zugbelastungen mehr auf das sich zwischen Wadenbein und Sprungbein erstreckende Band ausgeübt werden können. Ein Abwinkeln des Unterschenkels nach vorne wird dabei nicht behindert, so daß der Patient gehen kann, dabei aber aufgrund der Ausschaltung von sich auf das beschädigte Band auswirkenden Zugkräften keine störenden Schmerzen auftreten und der Heilungsprozeß nicht beeinträchtigt wird.

Die Haltebandage 1 umschließt den Vorder- oder Mittelfuß und kann vorzugsweise wie eine Klettverschlußverbindung angelegt und gespannt werden. Mit dieser Haltebandage 1 ist das sich in Richtung des Köchels erstreckende Zugband 2 vorzugsweise fest verbunden.

Die Haltebandage 1 muß bezüglich des Fußes fest positioniert sein, und zwar ohne die Blutzirkulation störende Verspannung. Erreicht wird dies beispielsweise durch eine Fixierung der Haltebandage mittels einer die Großzehe umschlingenden und mit der Haltebandage 1 fest verbundene Schlaufe, durch Verwendung eines zwischen Haltebandage 1 und Fuß angeordneten Klebebandes und bevorzugt durch Verwendung eines Formteils 5, das an die Fußkontur anmodellierbar ist und bevorzugt aus einem mit einer Gewebeeinlage versehenen dünnen Kunststofflage besteht. Dieses Formteil 5 umschließt den Fußaußenrand schalenförmig und liegt unterhalb der Haltebandage 1 und des Zugbands 2. Zwischen Haltebandage 1 und gegebenenfalls Zugband 2 sowie dem Formteil 5 besteht eine kraftschlüssige Verbindung, die beispielsweise durch Klettverschlußelemente, doppelseitige Klebebandabschnitte und dergleichen realisiert sein kann, so daß ein Fixpunkt bzw. ein Fixbereich an der Fußaußenseite geschaffen wird, der sicherstellt, daß ausgehend von diesem Bereich 6 das Zugband 2 definiert gespannt werden kann und die Richtung des Zugbandes 2 eindeutig festliegt.

Das vom Fixbereich 6 abgewandte Ende des Zugbandes kann mit der den Knöchelbereich umschlingenden Ringbandage vernäht sein, so daß die Spannung des Zugbandes 2 über die Ringbandage 3 erfolgt.

Möglich ist es jedoch auch, die Verbindung zwischen Ringbandage 3 und Zugband 2 über ein in sich geschlossenes Element, insbesondere ein Ringelement 4 vorzunehmen, da in diesem Falle das Zugband 2 für sich selbst noch spannbar ausgebildet sein kann, in dem das Zugbandende durch das Ringelement gezogen, zurückgeführt und mittels einer Klettverschlußverbindung nach Einstellung der gewünschten Spannung fixiert werden kann.

Die Ringbandage 3 besteht zweckmäßigerweise aus zwei über eine Klettverschlußverbindung aneinander fixierbaren und damit in der Gesamtlänge auch einstellbaren Teilen, wobei zur Erhöhung des Tragekomforts eine Abfütterung dieser Ringbandage 3 vorgesehen sein kann.

Entsprechend einer in der Zeichnung nicht dargestellten Ausführungsform kann für besondere Zwecke auch noch ein weiteres Zugband vorgesehen werden, das sich etwa vertikal nach unten erstreckt und damit zu einer zusätzlichen Fixierung führt. In diesem Falle wird die Verbindungsstelle zwischen dem Zugband und der Ringbandage 3 im Vergleich zu der in der Zeichnung dargestellten Position nach oben in den unmittelbaren Knöchelbereich verlegt, so daß es möglich ist, die dann vorhandenen beiden Zugbänder entsprechend den vom Wadenbein ausgehenden Bändern verlaufen zu lassen.

Die erfindungsgemäße Sprunggelenkbandage zeichnet sich somit durch besondere Einfachheit, hohe Wirksamkeit, leichte und korrekte Anlegbarkeit sowie dadurch aus, daß sie problemfrei auch in einem üblichen Schuh getragen werden kann.

## Patentansprüche

1. Bandage zur Fixierung des Sprunggelenks,
**gekennzeichnet** durch
eine den Mittelfußbereich umspannende Haltebandage (1) und ein mit dieser Haltebandage (1) fußaußenseitig verbundenes, sich zumindest im wesentlichen in Richtung des Außenknöchels erstreckendes Zugband (2), das mittels einer den Knöchelbereich umschlingenden, am Zugbandende angreifenden Ringbandage (3) spannbar ist.

2. Bandage nach Anspruch 1,
dadurch **gekennzeichnet,**
daß die Haltebandage (1) zumindest bereichsweise aus einem am Fuß haftenden Material besteht oder entsprechend haftend ausgerüstet ist.

3. Bandage nach Anspruch 1,
dadurch **gekennzeichnet,**
daß die Haltebandage (1) zumindest im fußaußenseitigen Bereich an einem den Fußaußenrand übergreifenden und unter der Haltebandage (1) gelegenen Formteil (5) fixierbar ist, das vorzugsweise aus einer der Fußform anpaßbaren, im wesentlichen formstabilen Materiallage besteht, die den Fußaußenrand flachbügelartig umgreift wobei die Haltebandage (1) mit dem Formteil (5) vorzugsweise über eine Klettverschluß- oder Haftklebeverbindung kuppelbar ist.

4. Bandage nach einem der vorhergehenden Ansprüche,
dadurch **gekennzeichnet,**
daß das Formteil (5) aus einer modellierbaren, insbesondere ein Gewebe einschließenden Kunststofflage besteht.

5. Bandage nach einem der vorhergehenden Ansprüche,
dadurch **gekennzeichnet,**
daß die Haltebandage (1) mittels einer eine oder mehrere Zehen umschließenden, insbesondere die Großzehe umschlingenden Schlaufe gehalten ist.

6. Bandage nach einem der vorhergehenden Ansprüche,
dadurch **gekennzeichnet,**
daß die Haltebandage (1) mittels eines doppelseitigen Klebebandes fußfest gehalten ist.

7. Bandage nach einem der vorhergehenden Ansprüche,
dadurch **gekennzeichnet,**
daß Haltebandage (1) und Zugband (2) einteilig ausgebildet sind.

8. Bandage nach einem der vorhergehenden Ansprüche,
dadurch **gekennzeichnet,**
daß die Haltebandage (1) über eine Klettverschlußverbindung verstellbar ist.

9. Bandage nach einem der vorhergehenden Ansprüche,
dadurch **gekennzeichnet,**
daß die Ringbandage (3) mit dem Zugband (2) unter Ausbildung einer Y-Bandverzweigung vorzugsweise über ein Ringelement (4) verbunden ist.

10. Bandage nach Anspruch 9,
dadurch **gekennzeichnet,**
daß die Ringbandage (3) mit dem Zugband (2) vernäht und das Zugband (2) insbesondere in seiner Länge verstellbar ausgebildet ist.

11. Bandage nach einem der vorhergehenden Ansprüche,
dadurch **gekennzeichnet,**
daß die Ringbandage (3) aus zwei über Verschlußorgane, insbesondere Klettverschlußorgane verbundenen, am Zugband (2) fixierten Bändern besteht und die Ringbandage (2) insbesondere zumindest auf einem Teilbereich ihres Umfangs gepolstert und / oder rückseitig verbreitert ausgeführt ist.

12. Bandage nach einem oder mehreren der vorhergehenden Ansprüche,
dadurch **gekennzeichnet,**
daß ein weiteres Zugband vorgesehen ist, das sich ausgehend von der Ringbandage (3) im wesentlichen vertikal nach unten erstreckt und fußaußenseitig analog dem ersten Zugband fixiert ist.
